# FASCICULE DE BREVET EUROPEEN

(11) **EP 0 680 722 B1**
(45) Date de publication et mention de la délivrance du brevet: **10.12.1997**
(21) Numéro de dépôt: 95400974.2
(22) Date de dépôt: 28.04.1995
(51) Int. Cl.: A61B 3/11

(54) **Procédé de métrologie optique**
Optisches Messtechnikverfahren
Process for optical measuring

(30) Priorité: 03.05.1994 FR 9405400
(43) Date de publication de la demande: 08.11.1995
(73) Titulaire: ESSILOR INTERNATIONAL Compagnie Générale d'Optique, F-94220 Charenton le Pont (FR)
(72) Inventeur: Ducarouge, Christian, F-69500 Lyon (FR); Grisel, Richard, F-69003 Lyon (FR); Giraud, Nicolas, F-69500 Lyon (FR); Sottocasa, Hélène, F-91370 Verrières (FR); Chansavoir, Alain, F-94100 Saint-Maur (FR); Haddadi, Ahmed, F-91210 Draveil (FR)
(74) Mandataire: Hirsch, Marc-Roger

(56) Documents cités:
- DE-U- 8 812 095
- FR-A- 2 538 239
- FR-A- 2 690 832

## Description

L'invention concerne un procédé de détermination des paramètres métrologiques d'un porteur de lunettes, à partir d'une image du porteur de lunettes muni d'une monture de lunettes, qui comprend les étapes consistant à déterminer sur ladite image la position des droites horizontales et verticales tangentes à la monture et la position des centres des pupilles du porteur, et à calculer à partir desdites positions les valeurs desdits paramètres métrologiques.

L'invention concerne le domaine technique de l'optique. Plus précisément, elle concerne la métrologie optique, c'est-à-dire la mesure des différents paramètres nécessaires à la réalisation des lunettes. Pour la réalisation de lunettes, il est nécessaire de tailler les lentilles destinées à être montées dans la monture, en fonction de la monture choisie et de différents paramètres liés au porteur des lunettes. Ces paramètres sont entre autres l'écart pupillaire, distance horizontale entre les yeux du porteur de lunettes, et les hauteurs ou décentrements.

Divers procédés de métrologie optique sont connus. De façon classique, l'opticien mesure directement les différents paramètres sur le porteur de la monture à l'aide d'un réglet. Cette méthode n'est pas très précise. Elle présente en outre l'inconvénient de ne pas mesurer la position naturelle du porteur des lunettes, mais une position sous contrainte: la tête est maintenue, différents dispositifs de mesure sont présents dans le champ visuel, etc.

Pour pallier ces inconvénients, il a été proposé dans le document FR-A-2 690 832 de saisir au moyen d'un dispositif de numérisation une image du visage d'un porteur muni d'une monture et de mesurer les divers paramètres sur cette image. Dans ce document, il est suggéré d'afficher cette image sur un écran. Ensuite, l'opticien repère sur l'écran avec une souris les points extrêmes haut et bas ainsi que latéraux de la monture, de façon à tracer sur l'écran un cadre rectangulaire tangent à la monture. La comparaison des dimensions de ce cadre à la forme théorique de la monture, obtenue par ailleurs, permet d'étalonner l'image. On repère ensuite sur cette image la position de la pupille de chaque oeil pour calculer les différents paramètres.

Dans un dispositif commercialisé par la firme Carl Zeiss sous la dénomination VIDEO-INFRAL, un procédé analogue est mis en oeuvre. A l'aide de deux caméras vidéo et d'un miroir, on prend des images du porteur de la monture, de face et de côté. Sur ces images, l'opticien trace avec une souris les tangentes à la monture et déplace ensuite le curseur ou la souris de façon à déterminer la position du centre de l'oeil. On mesure sur l'image prise de face la position des pupilles du patient par rapport à la monture, et sur l'image prise de côté, on repère l'inclinaison de la monture par rapport à la verticale.

Dans ces deux procédés, il est nécessaire que l'opticien trace avec une souris différents points de la monture et s'assure visuellement du parallélisme des tangentes. Ceci rend ces procédés longs à mettre en oeuvre. De plus, les tracés nécessaires diminuent la précision de la mesure; en effet, l'appréciation de la tangence des cadres par rapport à la monture dépend du manipulateur. Ces procédés fournissent difficilement des résultats reproductibles lors de mesures successives.

Dans un dispositif commercialisé par la firme Rodenstock sous la dénomination VIDEOCOM, il est proposé d'utiliser un simple caméscope, pour obtenir une image du porteur de la monture, après avoir adapté sur cette monture un accessoire millimétré. La mesure se fait ensuite en cliquant sur l'écran les points recherchés de cette image. Dans ce dispositif, la mesure est donc peu précise.

L'invention permet de pallier ces inconvénients. Elle assure une mesure reproductible, fiable, indépendante de l'opérateur. Elle permet de limiter les interventions de l'opérateur. Elle peut être entièrement automatisée, et fournir des mesures précises, quelle que soit la monture choisie.

L'invention est particulièrement adaptée à être mise en oeuvre avec le dispositif décrit dans le document FR-A-2 690 833, commercialisé par la demanderesse sous la dénomination VIDEOCENTRON.

L'invention a pour objet un procédé de détermination des paramètres métrologiques d'un porteur de lunettes, à partir d'une image du porteur de lunettes muni d'une monture de lunettes, tel que décrit plus haut, qui comprend les étapes consistant à:
- déterminer sur l'image, de façon automatique, la position du centre de chaque pupille par analyse du gradient de la luminance dans la zone de chaque pupille, et
- déterminer sur l'image, de façon automatique, les positions des droites horizontales et verticales tangentes à la monture, par analyse du gradient de luminance et extraction des contours de la monture.

L'invention limite les interventions humaines et fournit un résultat reproductible.

Dans un mode de mise en oeuvre du procédé, on détermine sur l'image les positions des droites horizontales et verticales tangentes au contour intérieur de chaque demi-monture.

Le procédé est plus précis que la recherche connue sur l'iamge des tangentes à la monture.

L'étape consistant à déterminer sur l'image, de façon automatique, la position du centre de chaque pupille peut comporter les étapes consistant, pour chaque pupille, à:
- déterminer une fenêtre couvrant la zone de la pupille;
- déterminer dans ladite fenêtre les points de l'image dont la luminance a une valeur supérieure à une valeur seuil;
- calculer l'isobarycentre desdits points.

Dans ce cas, l'étape consistant à déterminer ladite fenêtre peut être effectuée en recherchant dans la zone de chaque pupille le point de l'image dont la norme du gradient est maximale, et en centrant sur ce point une fenêtre de taille prédéterminée.

L'utilisation d'une telle fenêtre permet d'accélerer la mise en oeuvre du procédé.

Le procédé peut comprendre une étape consistant à définir autour de la position du centre de chaque pupille quatre fenêtres de taille prédéterminée, contenant chacune une droite tangente au contour intérieur de chaque demi-monture.

L'utilisation de telles fenêtres permet d'accélerer la mise en oeuvre du procédé, et de ne rechercher qu'une tangente dans chaque fenêtre.

L'étape consistant à déterminer sur l'image, de façon automatique, les positions des droites horizontales et verticales tangentes à la monture peut comprendre les étapes consistant, dans chacune desdites fenêtres à:
- déterminer dans ladite fenêtre les points de l'image dont la norme du gradient de la luminance est supérieure à une valeur seuil pour obtenir les contours intérieur et extérieur de la monture dans ladite fenêtre;
- déterminer la forme de la monture dans ladite fenêtre à partir desdits contours;
- déterminer en fonction de ladite fenêtre, la tangente au contour intérieur de la monture.

Avantageusement, ladite valeur de seuil est choisie de telle sorte qu'un nombre prédéterminé de points de l'image présente une valeur de norme de gradient supérieure audit seuil.

On peut calculer à partir de l'image les angles d'inclinaison, le procédé comportant les étapes consistant à:
- fournir au moins un contour réel de la monture;
- calculer pour les diverses valeurs possibles des angles d'inclinaison, la projection dudit contour réel sur l'image;
- comparer ladite projection avec les contours extraits de l'image en calculant, pour lesdites valeurs des angles d'inclinaison, la corrélation entre ladite projection et lesdits contours extraits de l'image;
les valeurs des angles d'inclinaison étant celles pour lesquelles ladite corrélation est maximale.

On peut aussi mettre en oeuvre l'invention en connaissant les angles d'inclinaison, le procédé comportant alors les étapes consistant à:
- fournir au moins un contour réel de la monture, et les angles d'inclinaison de la monture;
- calculer en fonction desdits angles d'inclinaison, la projection dudit contour réel sur l'image;
- comparer ladite projection avec les contours extraits de l'image en balayant ladite projection sur l'image et en calculant, en chaque position de ladite projection, la corrélation entre ladite projection et lesdits contours extraits de l'image;
- déterminer les positions des droites horizontales et verticales tangentes à la monture à partir de la position de ladite projection pour laquelle la dite corrélation est maximale.

Selon l'invention, l'étape consistant à calculer à partir desdites positions les valeurs desdits paramètres métrologiques peut s'effectuer en comparant les positions relatives des centres des pupilles et des droites tangentes aux contours intérieurs de la monture.

D'autres caractéristiques et avantages de l'invention apparaîtront à la lecture de la description qui suit d'un mode de réalisation de l'invention, donné à titre d'exemple et en référence aux dessins annexés qui montrent:
- figure 1 une représentation schématique des différents paramètres métrologiques d'un porteur de lunettes;
- figure 2 un ordinogramme des différentes étapes de détermination sur l'image de la position du centre de chaque pupille du porteur;
- figure 3 un ordinogramme des différentes étapes de détermination sur l'image de la position des tangentes verticales et horizontales;
- figure 4 une vue schématique de l'image d'une demi-monture avec les fenêtres de traitement;
- figure 5 un mode de détermination selon l'invention des tangentes horizontales et verticales;
- figure 6 un autre mode de détermination selon le procédé des tangentes horizontales et verticales;
- figure 7 un ordinogramme d'un autre mode de réalisation du procédé;

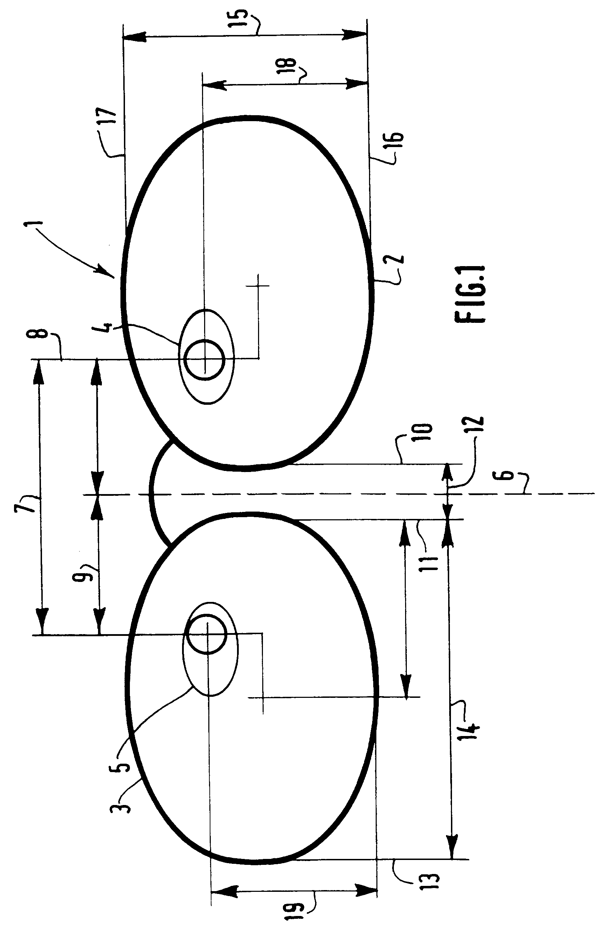

La figure 1 montre une représentation schématique des différents paramètres métrologiques d'un porteur de lunettes. Apparaissent sur la figure 1, une monture de lunettes 1 comprenant une demi-monture gauche 2 et une demi-monture droite 3, et des schémas des yeux gauche 4 et droit 5 du porteur de la monture. Pour la préparation de verres ou lentilles adaptés au porteur des lunettes et à la monture choisie, il est nécessaire de connaître la forme intérieure de la monture, pour la taille du verre. Il est aussi nécessaire de connaître la position par rapport à la monture des yeux du porteur. On utilise couramment les paramètres suivants. On appelle écart la distance entre les centres des deux pupilles du patient. L'écart est égal à la somme d'un demi-écart droit et d'un demi-écart gauche, correspondant respectivement aux distances horizontales entre la pupille droite et l'axe du nez et entre l'axe du nez et la pupille gauche. Sur la figure 1, l'axe du nez est représenté par le trait pointillé 6, l'écart par la cote 7, et les demi-écarts droit et gauche par les cotes 9 et 8. On appelle pont la distance entre les deux droites (10, 11) verticales tangentes à l'intérieur de chaque demi-monture du côté du nez. Le pont est repéré sur la figure 1 par la cote 12. On appelle A (cote 14) la largeur de chaque demi-monture, c'est-à-dire la distance entre les deux droites verticales 11 et 13 tangentes à l'intérieur de chaque demi-monture. On appelle B (cote 15) la distance séparant deux droites horizontales 16 et 17 tangentes à l'intérieur de chaque demi-monture. On appelle centre de chaque demi-monture l'intersection de la médiatrice des deux droites tangentes horizontales et de la médiatrice des deux droites tangentes verticales. On appelle HG ou hauteur gauche 18 (respectivement HD ou hauteur droite 19) la distance entre le centre de la pupille de l'oeil gauche 4 (respectivement droit 5) et la droite horizontale 16 tangente au bas de l'intérieur de la demi-monture gauche 2 (respectivement droite 3). On appelle décentrement horizontal ou vertical, gauche ou droit, la distance horizontale ou verticale entre le centre de chaque demi-monture et le centre de la pupille de l'oeil correspondant. On appelle hauteur sous pupille (droite ou gauche) la distance verticale entre le centre de la pupille (droite ou gauche) et le contour intérieur de la monture sous ce centre. On appelle rayon maximal (droit ou gauche) la distance maximale entre le centre de la pupille (droite ou gauche) et le contour intérieur de la monture correspondante. Le rayon maximal permet de déterminer le diamètre minimal de la lentille à utiliser.

Le but de l'invention est de mesurer ces différents paramètres à partir d'une image d'un porteur de lunettes muni d'une monture. Cette image est avantageusement une image obtenue par une caméra vidéo, couplée à une carte à numériser; une telle image peut par exemple fournie par le dispositif VIDEOCENTRON mentionné plus haut. Elle peut aussi être obtenue par un appareil photographique numérique, un scanner ou tout autre moyen. Une telle image est étalonnée de façon connue en soi par exemple par la présence dans l'image d'une mire de taille connue, ou par tout autre moyen permettant de déterminer le grandissement de l'image (connaisance de la distance focale, etc).

L'image est une image numérique en couleurs ou en niveaux de gris, sur laquelle apparaissent le visage du patient et la monture. Apparaît aussi sur cette image, lorsque le porteur des lunettes regarde une source lumineuse, un reflet au niveau de la cornée de chaque oeil.

Par image, on entend dans toute la suite une matrice de nombres, constituée d'un ensemble de points ou pixels. Dans le cas d'une image obtenue par une caméra vidéo, on travaille sur l'image de luminance ou sur tout type de représentation numérique de l'image (hue saturation intensity (HSI) ou red green blue (RGB)).

On entend par gradient la fonction mathématique usuelle, dans son application connue aux images discrètes. On peut aussi utiliser au lieu du gradient habituel toute fonction représentative des variations d'un champ de nombre, comme par exemple le laplacien.

On entend par norme la norme euclidienne classique. On peut aussi utiliser toute autre norme, ou encore une fonction qui n'est pas une norme au sens mathématique du terme, comme par exemple une simple somme des valeurs absolues des composantes, ou encore le carré de la norme euclidienne.

On utilise classiquement les termes d'abscisse et d'ordonnée pour les coordonnées horizontale et verticale.

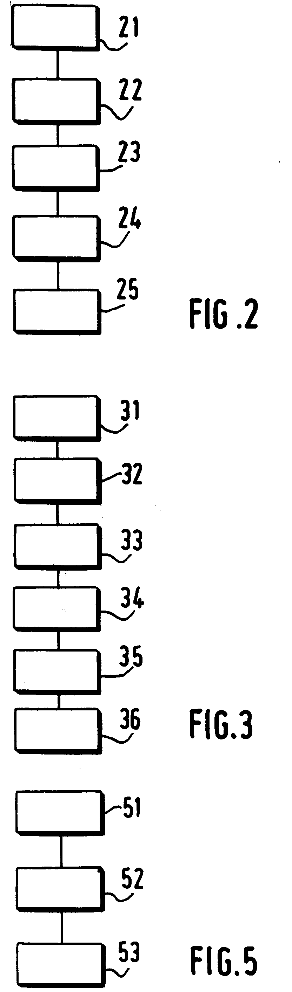

La figure 2 montre un ordinogramme des différentes étapes de détermination sur l'image de la position du centre de chaque pupille du porteur. Selon l'invention, la position du centre de chaque pupille est déterminée de façon automatique par analyse du gradient de luminance de l'image. Le reflet cornéen est d'abord détecté automatiquement par le calcul du pixel de norme de gradient de luminance maximale. Après un seuillage de l'information de luminance, le centre du reflet est ensuite déterminé par un calcul de barycentre. Le calcul peut se faire successivement sur chaque pupille ou en parallèle. L'invention est décrite en référence à la figure 2 pour un oeil.

L'ordinogramme de la figure 2 montre un mode de réalisation plus détaillé. A l'étape 21, on détermine une fenêtre de détection pour la recherche du centre de la pupille. Dans un appareil donné, pour une position donnée du porteur de la monture, la position d'un oeil sur l'image varie relativement peu en fonction des individus. La fenêtre de détection est située autour de la position prévue de l'oeil, et présente une dimension déterminée experimentalement pour un appareil donné; cette dimension est telle que l'image de l'oeil se trouve dans la fenêtre, quel que soit le porteur.

L'utilisation de cette fenêtre au lieu de travailler sur toute l'image permet de réduire le temps de calcul. On pourrait aussi travailler sur l'ensemble de l'image .

A l'étape 22, on calcule dans la fenêtre déterminée à l'étape 21, la norme du gradient de luminance en tout point.

A l'étape 23, on recherche dans la fenêtre la frontière du reflet cornéen sur la pupille, c'est-à-dire le pixel présentant la norme de gradient maximale. On centre, sur ce pixel, une nouvelle fenêtre de traitement de taille plus réduite. Cette fenêtre, pour un appareil donné, pour une position donnée du porteur des lunettes, a une taille déterminée expérimentalement pour comprendre seulement le reflet sur la pupille. On obtient ainsi une fenêtre de taille plus réduite. On effectue ensuite une binarisation: tous les pixels dont la valeur de luminance est supérieure à une valeur donnée sont mis à un, les autres à zéro.

A l'étape 24, on élimine les reflets parasites en effectuant une érosion morphologique sur la fenêtre binarisée. L'érosion morphologique consiste à comparer la valeur du pixel de l'image avec les valeurs des pixels voisins. On affecte à chaque pixel la valeur minimum rencontrée sur un voisinage de pixels déterminé. Ainsi, les reflets de taille inférieure au voisinage disparaissent.

A l'étape 25, on calcule, dans la fenêtre de l'étape 23, l'isobarycentre des pixels retenus. Le point ainsi obtenu est le centre du reflet cornéen et correspond au centre de la pupille en cause. On pourrait aussi calculer le barycentre des pixels affectés des masses correspondant à la valeur de luminance, dans ce cas sans nécessairement effectuer de seuillage et de binarisation.

On applique de façon analogue les étapes 21 à 25 pour déterminer la position du centre de la deuxième pupille.

On obtient ainsi la position du centre de chaque pupille du porteur dans l'image. A l'encontre des méthodes connues, il n'est pas nécessaire pour l'opérateur d'acquérir la position de la pupille du porteur, à l'aide d'une souris. La détermination obtenue est aussi plus précise qu'une simple acquisition manuelle. On calcule ainsi de façon automatique la valeur de l'écart, en effectuant la différence entre la abscisses des centres déterminés.

La figure 3 montre un ordinogramme des différentes étapes de détermination sur l'image de la position des tangentes verticales et horizontales. Selon l'invention, on détermine les droites horizontales et verticales tangentes à l'intérieur de la monture. On évite ainsi les problèmes de l'art antérieur liés à l'épaisseur de la monture et à l'interprétation des couleurs des montures.

L'invention propose de déterminer sur l'image les contours intérieurs et extérieurs de la monture, par analyse du gradient de luminance. On commence par calculer en tout point la norme du gradient de luminance. On applique ensuite un seuillage, autrement dit on binarise le champ ainsi obtenu. De la sorte, on détermine les contours intérieur et extérieur de chaque demi-monture. La valeur de seuillage peut avantageusement être déterminée en calculant l'histogramme cumulé du gradient. On détermine ensuite la forme complète de la monture en appliquant une fermeture morphologique. Ceci permet ensuite de déterminer la tangente intérieure à la monture.

La figure 3 montre un ordinogramme plus détaillé de cette partie du procédé selon l'invention, pour une demi-monture. Comme expliqué en référence à la figure 2, le calcul peut se faire successivement sur chaque demi-monture ou en parallèle. L'invention est décrite en référence à la figure 3 pour une demi-monture.

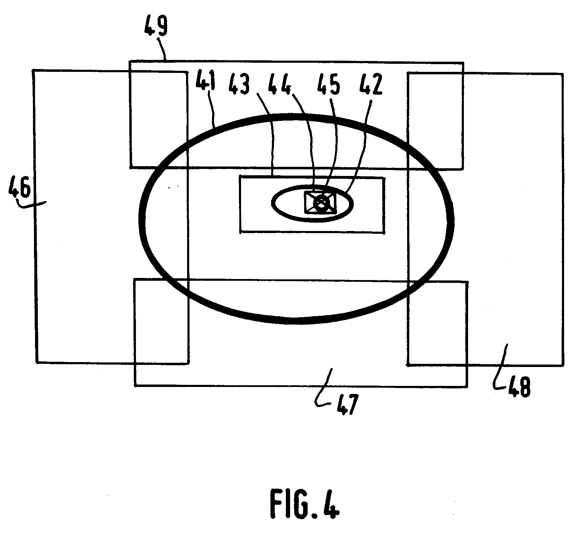

A l'étape 31, on détermine quatre fenêtres de traitement (gauche, droite, haut et bas), positionnées par rapport au centre de la pupille obtenu comme expliqué en référence à la figure 2. En effet, pour accélérer la vitesse du traitement numérique, on détermine la position des tangentes dans quatre fenêtres de traitement et non pas sur l'ensemble de l'image, comme cela apparaît de la description de la figure 4. Pour un appareil donné et une position donnée du porteur des lunettes, la taille et la position relative de ces quatre fenêtres sont déterminées expérimentalement, de façon à ce que ces quatre fenêtres contiennent chacune une tangente, pour toutes formes de monture. Les quatre fenêtres sont disposées sur l'image par rapport au centre de la pupille déjà déterminé.

A l'étape 32, dans chacune des fenêtres, on calcule la carte de la norme du gradient de luminance.

A l'étape 33, on détermine une valeur de seuillage. Cette valeur peut être une valeur fixe, déterminée expérimentalement pour un appareil de prise de vue donné; toutefois, elle peut aussi avantageusement être déterminée comme suit. A partir de la carte de la norme du gradient de luminance, on calcule un histogramme cumulé: autrement dit, on calcule pour chaque valeur possible de la norme du gradient, le nombre de pixels de l'image pour lesquels la norme du gradient présente ladite valeur. On obtient ainsi une idée de la répartition de la norme du gradient sur l'image. On choisit comme valeur de seuillage une valeur telle qu'un nombre déterminé de points présentent une norme de gradient supérieure audit seuil. Par exemple, dans une fenêtre de 150 x 80 pixels, on peut retenir 2 000 pixels. Ce nombre de pixels est le nombre de pixels constituant les contours de la monture.

A l'étape 34, on sélectionne les points dont la norme du gradient est supérieure à la valeur de seuil de l'étape 33. On obtient ainsi dans chaque fenêtre les contours intérieur et extérieur de la monture.

A l'étape 35, on précise la forme des contours obtenus à l'étape 34, par un amincissement des contours. A cette étape, on travaille dans chaque fenêtre dans une direction privilégiée. Ainsi, dans les deux fenêtres latérales, on recherche une tangente verticale à la monture, et la direction privilégiée est la direction verticale; inversement, dans les deux fenêtres haut et bas, on recherche une tangente horizontale à la monture, et la direction privilégiée est la direction horizontale. Ceci apparaît plus clairement de la description de la figure 4. On effectue dans chaque fenêtre une érosion dans une direction orthogonale à la direction privilégiée. On obtient ainsi une image plus précise des contours intérieur et extérieur de la monture. Autrement dit, par exemple dans la fenêtre gauche, on effectue une érosion dans une direction horizontale, par exemple en comparant la valeur de la norme de chaque pixel à celle de ses deux voisins horizontaux, et en ne conservant que celui dont la norme du gradient est la plus importante.

A l'étape 36, on binarise les contours obtenus. On obtient ainsi une image binaire qui, dans chaque fenêtre, contient les contours intérieur et extérieur de la monture.

De façon analogue à ce qui a été décrit à la figure 2, on effectue un traitement similaire sur chaque demi-monture, successivement ou en parallèle.

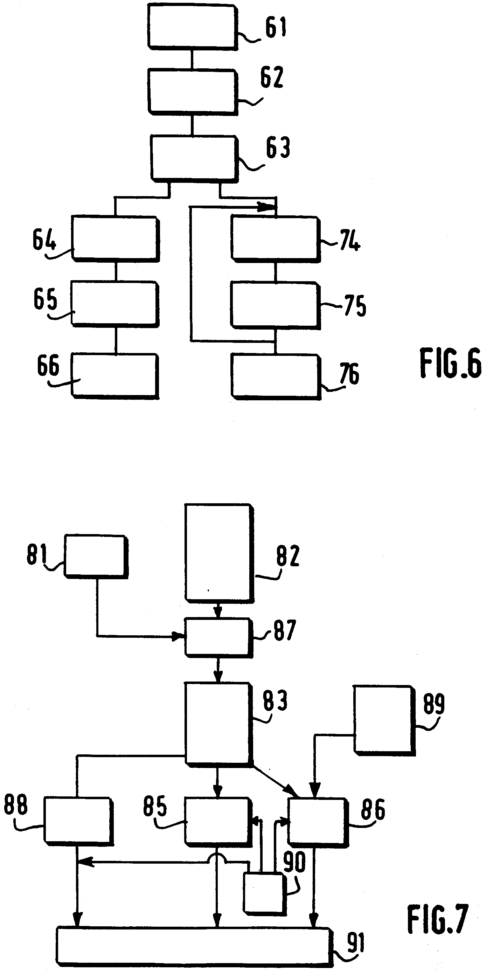

Sur l'image obtenue à l'étape 36, on peut soit effectuer un traitement direct, soit effectuer un traitement à l'aide d'un fichier de monture prédéterminé. Ces deux traitements sont décrits en référence aux figures 5 et 6.

La figure 4 montre une vue schématique de l'image d'une demi-monture avec les fenêtres de traitement décrites en références aux figures 2 et 3. On voit sur la figure 4 une demi-monture 41, une représentation schématique d'un oeil 42; la référence 43 indique la fenêtre de détection de l'étape 21 de la figure 3; la référence 44 indique la fenêtre de détection de taille plus réduite de l'étape 24 de la figure 2. Le cercle 45 représente schématiquement le contour de la pupille ou du reflet cornéen. Les références 46 à 49 indiquent les quatre fenêtres de détection mentionnées à la figure 3, dans lesquelles on cherche les différentes tangentes au contour intérieur de la monture.

La figure 5 illustre un mode de détermination selon l'invention des tangentes horizontales et verticales. Par détemination des tangentes horizontales et verticales, on n'entend pas nécessairement le tracé de ces tangentes, mais simplement la détermination de leur position, soit dans l'image, soit simplement par rapport au centre de la pupille. Ainsi, si l'on parle de détemination d'une tangente horizontale, il faut comprendre la détermination soit de l'ordonnée de cette tangente, soit la détermination de la distance verticale qui la sépare du centre de la pupille (ce qui revient au même). Dans le mode de la figure 5, on n'utilise pas de fichiers externes représentatifs de la forme de la monture. Comme décrit plus haut, on effectue les étapes 51 et 52 sur chaque demi-monture, en parallèle ou successivement.

A l'étape 51, on traite dans chaque fenêtre les contours intérieur et extérieur obtenus à l'étape 36, de façon à effectuer une fermeture morphologique pour "remplir" la monture entre le contour intérieur et le contour extérieur. On obtient ainsi dans chaque fenêtre une image non plus des contours de la monture mais de la monture elle-même. La fermeture morphologique s'effectue par dilatation puis érosion successives. La dilatation consiste en partant de l'image des contours, à noircir chaque pixel voisin d'un pixel d'un des contours. On "épaissit" ainsi chaque contour. On effectue cette étape éventuellement plusieurs fois de sorte que les contours intérieur et extérieur se rejoignent. On effectue ensuite une opération inverse d'érosion morphologique qui diminue la taille des contours. Cette opération d'érosion est, le cas échéant, répétée autant de fois que l'opération de dilatation. On obtient ainsi une image de la monture. Cette dilatation et cette érosion morphologiques peuvent se faire dans une direction privilégiée dans chaque fenêtre, suivant le principe exposé en réference à l'étape 35 de la figure 3.

A l'étape 52, on effectue sur l'image de la monture, dans chaque fenêtre, la recherche d'une tangente au contour intérieur. L'invention permet de rechercher avec précision la droite tangente au contour intérieur. Elle est plus précise que l'appréciation "à l'oeil" d'un opérateur, qui ext utilisée dans l'art antérieur. Pour tracer la droite tangente, on peut par exemple utiliser une méthode d'approximation de droite. Cette méthode consiste à rechercher dans la monture le segment de droite le plus long, dans une direction donnée. Ainsi, par exemple dans la fenêtre haute (référence 49 sur la figure 4), on recherche une tangente horizontale. En partant du bas de la fenêtre (i.e. du côté intérieur de la monture), on détermine la longueur du segment horizontal le plus long de la monture, en prenant en compte les segments précédemment rencontrés et les recouvrements. A longueur égale, on retient le segment le plus bas, i.e. le plus proche de l'intérieur de la monture. On obtient ainsi la tangente horizontale au contour intérieur de la monture.

On pourrait utiliser une autre méthode de recherche de tangente, comme par exemple une méthode de suivi de contour. Dans ce cas, il ne serait pas forcément nécessaire d'effectuer l'étape 51, utile dans le cas d'une méthode d'approximation de droite. On pourrait déterminer les tangentes directement à partir des contours intérieur et extérieur.

A l'issue de l'étape 52, on connaît dans l'image la position des droites horizontales et verticales tangentes à l'intérieur de chaque demi-monture et on connaît la position des centres des pupilles du porteur. On détermine ainsi la valeur en pixels de la projection des paramètres métrologiques sur l'image. On calcule en pixels la hauteur sous pupille en considérant le point du contour intérieur ayant la même abscisse que le centre de la pupille. On calcule en pixels le rayon maximal en calculant pour tous les points du contour intérieur la distance au centre de la pupille et en cherchant le maximum de cette distance sur le contour intérieur.

Connaissant le grandissement de l'image, on détermine en millimètres la valeur de la projection des paramètres métro-logiques.

La connaissance des angles d'inclinaison de la monture par rapport au plan de l'image permet de calculer les différents paramètres métrologiques. En effet, la monture, du fait que le port de la tête est libre, peut avoir subi un basculement latéral (un côté de la monture est plus haut que l'autre). Elle peut aussi être décalé par rapport au plan de l'image, un côté de la monture étant plus en "avant" que l'autre: ceci est le cas si le porteur de la monture ne regarde pas en face la caméra vidéo ou analogue, ou tourne légèrement la tête. Enfin, la monture n'est pas généralement portée dans un plan vertical. Le plan de la monture fait, avec un plan vertical, un angle appelé angle pantoscopique. Connaissant les valeurs de ces trois angles et la valeur de la projection des paramètres métrologiques sur l'image, on peut, à l'étape 53, calculer les valeurs en millimètres des paramètres métrologiques A, B, hauteurs et décentrements; connaissant l'écart (voir plus haut fig. 2), on calcule le pont, et les demi-écarts gauche et droit. On calcule aussi la hauteur sous pupille et le rayon maximal.

La figure 6 montre un autre mode de détermination selon le procédé des tangentes horizontales et verticales. Dans le mode de réalisation de la figure 6, on utilise un fichier externe représentatif de la forme du contour interne de la monture portée par le porteur de lunettes sur l'image (monture réelle ou contour réel). Un tel fichier peut, par exemple, être programmé dans un dispositif de mise en oeuvre de l'invention. Il peut être fourni par un lecteur de monture, à palpeur, du type de celui comme réalisé par la demanderesse sous la dénomination DIGISCAN. Il peut aussi être obtenu par prise d'une image vidéo sur fond discriminant ou par tout autre moyen. Dans le cas où le fichier est représentatif de la forme de la monture au fond du drageoir, il est corrigé de façon à obtenir un fichier représentatif du contour intérieur de la monture (par exemple par érosion vers l'intérieur d'une quantité égale à la profondeur du drageoir).

Ce fichier est utilisé selon l'invention pour affiner et accélérer le calcul des tangentes. Pour cela, l'invention propose de caler par le calcul le contour réel sur l'image obtenue en tenant compte des angles d'inclinaison de la monture et du grandissement de l'image. Lorsque le contour réel se superpose à la monture obtenue sur l'image, on calcule les tangentes. En tenant compte des angles d'inclinaison, on détermine alors les paramètres géométriques. Le calcul des tangentes est simplifié, car on connaît avec précision les points extrêmes du contour réel.

Cette partie du procédé évite la mesure des angles d'inclinaison. En effet, on peut par le calcul simuler divers angles d'inclinaison sur le contour réel, et comparer avec la monture obtenue sur l'image. La corrélation maximale entre le contour réel et la monture de l'image fournit les angles d'inclinaison recherchées.

La figure 6 montre un mode de réalisation détaillé. Comme plus haut, on travaille sur chaque demi-monture, ou sur les deux demi-montures à la fois.

A l'étape 61, on lit le fichier représentatif de la monture. Il peut par exemple s'agir d'un simple fichier binaire. Ce fichier, si nécessaire, subit une homothétie de façon à être adapté au grandissement de l'image.

A l'étape 62, on effectue une vectorisation de la forme de la monture, pour diminuer le nombre de points la définissant et faciliter les calculs. On pourrait à l'étape 62 employer une autre méthode de compression, par exemple par interpolation de courbes de Béziers, ou par tout autre fonction. On obtient ainsi une image du contour intérieur de la monture (contour réel).

A l'étape 63, on fait subir au contour réel ainsi obtenu une érosion pour diminuer les erreurs d'acquisition et préciser le contour interne de la monture. Le cas échéant, le contour réel est traité pour qu'il représente le contour intérieur de la monture (cas du drageoir).

On peut ensuite mettre en oeuvre plusieurs modes de réalisation du procédé. Si l'on connaît les différents angles d'inclinaison, on peut procéder comme aux étapes 64 à 66. Sinon, on peut procéder comme aux étapes 74 à 76.

A l'étape 64, à partir des angles d'inclinaison, on calcule la projection du contour réel de la monture sur l'image.

A l'étape 65, on balaye sur l'image la projection ainsi calculée. Autrement dit, on déplace sur l'image la projection du contour réel. On calcule en chaque position la corrélation (recouvrement ou nombre de points communs) entre la projection du contour réel de la monture et la monture identifiée sur l'image. Lorsque cette corrélation est maximale, la position du contour réel donne la position de la monture.

A l'étape 66, on calcule, connaissant la position de la monture, les positions des différentes tangentes. La position des différentes tangentes peut être déterminée simplement connaissant les points extrêmes du contour réel (maxima en abscisse et en ordonnée). On peut le cas échéant appliquer une méthode d'approximation de droite au voisinage de ces points. Dans ce cas, il suffit d'appliquer cette méthode dans une fenêtre plus petite.

On peut ensuite calculer, comme à l'étape 53, les différents paramètres métrologiques A, B, hauteurs et décentrement; connaissant le fichier monture, on détermine directement le pont, ou l'on procède comme décrit plus haut.

Alternativement, si l'on ne connaît pas les différents angles d'inclinaison, on peut procéder par itérations (étapes 74 à 76).

A l'étape 74, on calcule pour des angles d'inclinaison donnés, la projection du contour réel de la monture sur l'image.

A l'étape 75, on balaye cette projection sur l'image et on détermine la corrélation maximale entre la projection et la monture obtenue à l'issue de l'étape 36. On stocke cette valeur de corrélation en liaison avec les valeurs correspondantes des angles d'inclinaison.

On repasse ensuite à l'étape 74, en changeant la valeur des angles d'inclinaison.

On continue à itérer de la sorte jusqu'à avoir balayé les plages possibles d'angles d'inclinaison (par exemple, basculement entre -3° et +3°, angle pantoscopique entre 0° et 15°, avec un balayage de 0,5° en 0,5°, soit 13 x 31 itérations). La corrélation maximale indique les bonnes valeurs d'angles d'inclinaison. On passe ensuite à l'étape 76.

A l'étape 76, on procédé comme à l'étape 66 pour calculer les paramètres métrologiques. On obtient ainsi les différents paramètres métrologiques à mesurer.

L'invention permet ainsi de tenir compte de la forme réelle de chaque monture et, en particulier, de son épaisseur, et fournit des résultats précis et reproductibles. Elle ne nécessite aucune intervention de l'opérateur. Elle peut être mise en oeuvre avec ou sans mesure des angles d'inclinaison.

La figure 7 montre un ordinogramme d'un autre mode de réalisation du procédé. Dans le mode de réalisation de la figure 7, l'opérateur peut intervenir et réaliser lui-même des mesures sur l'image. Ces mesures sont ensuite comparées avec celles obtenues comme décrit précédemment. A la figure 7, la référence 82 indique globalement les différentes étapes décrites en référence à la figure 2; la référence 83, 85 et 86 indiquent globalement les différentes étapes décrites en référence respectivement aux figures 3, 5 et 6.

A l'étape 82, on détermine sur l'image la position des pupilles du porteur de la monture. Le cas échéant, un opérateur peut, à l'aide d'un curseur ou d'une souris, déterminer aussi cette position, comme indiqué par la référence 81. A l'étape 87, les résultats sont comparés et la différence est affichée. La référence 88 indique un mode d'acquisition manuel des tangentes à la monture, comme dans les dispositifs connus. La référence 89 indique la source du fichier externe contenant la forme de la monture et la référence 90 indique un dispositif fournissant les angles d'inclinaison. La référence 91 montre l'affichage des paramètres géométriques.

Le procédé selon l'invention est ainsi susceptible de diverses variantes: il peut être utilisé en mode manuel (81, 83, 88, 90 et 91), l'opérateur ou l'opticien gardant alors le contrôle. Il peut être utilisé en mode automatique sans connaissance de la monture (82, 83, 85, 90 et 91). Il peut enfin être utilisé en mode automatique avec l'aide d'un fichier externe représentatif de la monture. On peut alors déterminer automatiquement les angles d'inclinaison (82, 83, 86, 89 et 91) ou encore utiliser des valeurs connues ou mesurées par ailleurs (82, 83, 86, 89, 90 et 91).

Le procédé permet de tenir compte des trois angles d'inclinaison possibles et de l'épaisseur de la monture. De ce seul fait, il fournit des résultats plus fiables et plus précis que les procédés de l'art antérieur.

Le procédé selon l'invention peut être mis en oeuvre dans tout dispositif fournissant une image d'un porteur d'une monture. Il est en particulier adapté à être mis en oeuvre dans le dispositif décrit dans le brevet FR-A-2 690 833 mentionné plus haut. Les caractéristiques décrites dans ce brevet et utiles pour la mise en oeuvre du procédé selon l'invention, en combinaison avec ledit procédé, doivent être considérées comme faisant partie de l'invention.

Bien entendu, l'invention n'est pas limitée aux modes de réalisation donnés à titre d'exemple. On pourrait ainsi déterminer les tangentes par rapport aux contours extérieurs de la monture, ou encore par rapport au squelette de la monture, si cela était nécessaire pour les opticiens. L'utilisation de fenêtres de traitement permet d'accélérer les calculs: elle n'est pas indispensable à la réalisation de l'invention. Pour étalonner l'image, on peut aussi, le cas échéant, lui faire subir une homothétie. On pourrait aussi, pour le calcul des angles d'inclinaison, utiliser d'autres méthodes, comme par exemple celle décrite dans le FR-A-2 690 832. Pour la détermination des tangentes, on peut utiliser d'autres algorithmes que les méthodes d'approximation de droite, et par exemple des méthodes de suivi de contour. On a décrit ci-dessus une analyse du gradient de luminance de l'image: on pourrait aussi travailler sur un autre type de représentation de l'image (RGB, HSI) et/ou avec un autre type d'opérateur mathématique (valeur directe du pixel, laplacien, ...). Dans l'analyse du contour réel de la monture, on a décrit une comparaison sur la base du contour intérieur de la monture: on peut aussi travailler sur le contour extérieur de la monture, ou sur les deux contours.

## Revendications

1. Procédé de détermination des paramètres métrologiques (7, 8, 9, 12, 14, 15, 18, 19) d'un porteur de lunettes, à partir d'une image du porteur de lunettes muni d'une monture de lunettes (1), comprenant les étapes consistant à:
- déterminer sur ladite image la position des droites horizontales et verticales tangentes à la monture et la position des centres des pupilles du porteur;
- calculer à partir desdites positions les valeurs desdits paramètres métrologiques,
caractérisé en ce qu'il comprend les étapes consistant à:
- déterminer sur l'image, de façon automatique, la position du centre de chaque pupille par analyse du gradient de la luminance dans la zone de chaque pupille, et
- déterminer sur l'image, de façon automatique, les positions des droites horizontales et verticales tangentes à la monture, par analyse du gradient de luminance et extraction des contours de la monture.

2. Procédé selon la revendication 1, caractérisé en ce que l'on détermine sur l'image les positions des droites horizontales et verticales tangentes au contour intérieur de chaque demi-monture.

3. Procédé selon la revendication 1 ou 2, caractérisé en ce que l'étape consistant à déterminer sur l'image, de façon automatique, la position du centre de chaque pupille comporte les étapes consistant, pour chaque pupille, à:
- déterminer une fenêtre (44) couvrant la zone de la pupille (42);
- déterminer dans ladite fenêtre les points de l'image dont la luminance a une valeur supérieure à une valeur seuil;
- calculer l'isobarycentre desdits points.

4. Procédé selon la revendication 3, caractérisé en ce qu'il comprend l'étape consistant à déterminer la dite fenêtre (44) s'effectue en recherchant dans la zone de chaque pupille le point de l'image dont la norme du gradient est maximale, et en centrant sur ce point une fenêtre de taille prédéterminée.

5. Procédé selon l'une des revendications 2 à 4, caractérisé en ce qu'il comprend une étape consistant à définir autour de la position du centre de chaque pupille quatre fenêtres (46, 47, 48, 49) de taille prédéterminée, contenant chacune une droite tangente au contour intérieur de chaque demi-monture.

6. Procédé selon la revendication 5, caractérisé en ce que l'étape consistant à déterminer sur l'image, de façon automatique, les positions des droites horizontales et verticales tangentes à la monture comprend les étapes consistant, dans chacune desdites fenêtres (46, 47, 48, 49) à:
- déterminer dans ladite fenêtre les points de l'image dont la norme du gradient de la luminance est supérieure à une valeur seuil pour obtenir les contours intérieur et extérieur de la monture dans ladite fenêtre;
- déterminer la forme de la monture dans ladite fenêtre à partir desdits contours;
- déterminer en fonction de ladite fenêtre, la tangente au contour intérieur de la monture.

7. Procédé selon la revendication 6, caractérisé en ce que ladite valeur de seuil est choisie de telle sorte qu'un nombre prédéterminé de points de l'image présentent une valeur de norme de gradient supérieure audit seuil.

8. Procédé selon l'une des revendications 1 à 7, caractérisé en ce qu'il comprend une étape de calcul des angles d'inclinaison, comportant les étapes consistant à:
- fournir au moins un contour réel de la monture;
- calculer pour les diverses valeurs possibles des angles d'inclinaison, la projection dudit contour réel sur l'image;
- comparer ladite projection avec les contours extraits de l'image en calculant, pour lesdites valeurs des angles d'inclinaison, la corrélation entre ladite projection et lesdits contours extraits de l'image;
les valeurs des angles d'inclinaison étant celles pour lesquelles ladite corrélation est maximale.

9. Procédé selon l'une des revendications 1 à 7, caractérisé en ce qu'il comporte les étapes consistant à:
- fournir au moins un contour réel de la monture, et les angles d'inclinaison de la monture;
- calculer en fonction des dits angles d'inclinaison, la projection du dit contour réel sur l'image;
- comparer ladite projection avec les contours extraits de l'image en balayant ladite projection sur l'image et en calculant, en chaque position de ladite projection, la corrélation entre ladite projection et lesdits contours extraits de l'image;
- déterminer les positions des droites horizontales et verticales tangentes à la monture à partir de la position de ladite projection pour laquelle ladite corrélation est maximale.

10. Procédé selon l'une des revendications 1 à 9, caractérisé en ce que l'étape consistant à calculer à partir desdites positions les valeurs desdits paramètres métrologiques s'effectue en comparant les positions relatives des centres des pupilles et des droites tangentes aux contours intérieurs de la monture.

## Claims

1. A method for determining measurement parameters (7, 8, 9, 12, 14, 15, 18, 19) for a spectacle wearer from an image of said spectacle wearer provided with a spectacle frame (1) comprising the steps of
- determining, on said image, the position of the horizontal and vertical straight lines tangential to said frame and the position of the centers of the pupils of the eyes of said wearer;
- and calculating the values of said measurement parameters from said positions,
characterized in that it includes the steps of:
- automatically determining, on said image, the position of the center of each pupil by analysing a luminance gradient in the region of each pupil, and
- determining automatically, on said image, the positions of the horizontal and vertical straight lines tangential to said frame, by luminance gradient analysis and extraction of the contours of said frame.

2. Method according to claim 1, characterized in that it is the positions of the horizontal and vertical straight lines tangential to the inside contour of each half-frame that are determined on said image.

3. A method according to claim 1 or 2, characterized in that the step consisting of determining the position of the center of each pupil of the eye automatically on said image comprises the steps consisting, for each pupil, of:
- determining a window (44) covering the region of the pupil (42);
- determining, within said window, those point on the image having a luminance that is higher than a threshold value;
- calculating the position of the centroid of said points.

4. A method according to claim 3, characterized in that the step consisting in determining said window (44) is done by seeking, in the region of each pupil, that point in the image for which the norm of the gradient is a maximum, and then centering a window of a predetermined size on said point.

5. Method according to one of claims 2 to 4, characterized in that it includes a step consisting in defining four windows (46, 47, 48, 49) of predetermined size around the position of the center of each pupil, each containing one straight line tangential to the inside contour of each half-frame.

6. Method according to claim 5, characterized in that the step consisting in determining the positions of the horizontal and vertical straight lines tangential to said frame automatically comprises the steps, within each of said windows (46, 47, 48, 49), of:
- determining, within said window, those points on the image for which the norm of the luminance gradient is higher than the threshold value in order to obtain the inside and outside contours of said frame within said window;
- determining the shape of said frame within said window, from said contours;
- determining, as a function of said window, the tangent to the inside contour of said frame.

7. Method according to claim 6, characterized in that said threshold value is selected whereby a predetermined number of said points on the image have a value of the norm of their luminance gradient above said threshold value.

8. Method according to one of claims 1 to 7, characterized in that it includes a step consisting in calculating angles of inclination comprising the steps of:
- supplying at least a real contour of said frame;
- calculating, for the various possible values of angles of inclination, the projection of said real contour on said image;
- comparing said projection with the contours extracted from said image by calculating, for said values of angles of inclination, the correlation between said projection and said contours extracted from said image;
the values of said angles of inclination being those values for which said correlation is at a maximum.

9. Method according to one of claims 1 to 7, characterized in that it comprises the steps of:
- supplying at least a real contour of said frame, and the angles of inclination of said frame;
- calculating, as a function of said angles of inclination, the projection of said real contour on said image;
- comparing said projection with the contours extracted from said image by sweeping said projection over said image and calculating therefrom, for each position of said projection, the correlation between said projection and said contours extracted from said image;
- determining the positions of the horizontal and vertical straight lines tangential to said frame, using the position of said projection for which said correlation is at a maximum.

10. Method according to one of claims 1 to 9, characterized in that the step consisting in calculating the values of said measurement parameters from said positions is achieved by comparing the relative positions of the centers of the pupils of the eyes of said spectacle wearer and the straight lines tangential to the inside contours of said frame.

## Patentansprüche

1. Verfahren zur Bestimmung der metrologischen bzw. meßtechnischen Parameter (7, 8, 9, 12, 14, 15, 18, 19) eines Brillenträgers, ausgehend von einem Bild des Brillenträgers, welches mit einer Brillenfassung (1) versehen ist, umfassend die Schritte bestehend aus:
- Bestimmen der Positon der Tangenten an die Brillenfassung bildenden, horizontalen und vertikalen Geraden und der Position der Zentren der Pupillen des Trägers auf diesem Bild;
- ausgehend von diesen Positionen Berechnen der Werte dieser metrologischen Parameter,
dadurch gekennzeichnet, daß es die Schritte umfaßt, bestehend aus:
- einem automatischen Bestimmen auf dem Bild der Position des Zentrums von jeder Pupille durch Analyse des Helligkeitsgradienten im Bereich von jeder Pupille, und
- einem automatischen Bestimmen auf dem Bild der Positionen der Tangenten an die Brillenfassung bildenden, horizontalen und vertikalen Geraden durch Analyse des Helligkeitsgradienten und Abziehen der Konturen der Fassung.

2. Verfahren nach Anspruch 1, dadurch gekennzeichnet, daß auf dem Bild die Positionen der Tangenten an die Innenkontur von jeder Fassungshälfte bildenden, horizontalen und vertikalen Geraden bestimmt werden.

3. Verfahren nach Anspruch 1 oder 2, dadurch gekennzeichnet, daß der Schritt bestehend aus dem automatischen Bestimmen auf dem Bild der Position des Zentrums von jeder Pupille für jede Pupille die Schritte umfaßt:
- Bestimmen eines den Bereich der Pupille (42) abdeckenden Fensters (44);
- in diesem Fenster Bestimmen der Punkte des Bildes deren Helligkeit einen über einem Schwellwert liegenden Wert aufweisen;
- Berechnen des Isobarenzentrums bzw. Schwerpunkts dieser Punkte.

4. Verfahren nach Anspruch 3, dadurch gekennzeichnet, daß der Schritt, welcher im Bestimmen dieses Fensters (44) besteht, durch ein Suchen im Bereich von jeder Pupille des Punktes des Bildes, in welchem die Größe des Gradienten maximal ist und Zentrieren eines Fensters mit vorbestimmter Größe in diesem Punkt, ausgeführt wird.

5. Verfahren nach einem der Ansprüche 2 bis 4, dadurch gekennzeichnet, daß es einen Schritt bestehend aus dem Definieren von vier Fenstern (46, 47, 48, 49) mit vorbestimmter Größe rund um die Position des Zentrums von jeder Pupille umfaßt, welche Fenster jeweils eine eine Tangente an die Innenkontur von jeder Fassungshälfte bildende Gerade enthalten.

6. Verfahren nach Anspruch 5, dadurch gekennzeichnet, daß der Schritt bestehend in dem automatischen Bestimmen der Positionen der Tangenten an die Fassung bildenden horizontalen und vertikalen Geraden auf dem Bild in jedem der Fenster (46, 47, 48, 49) die Schritte umfaßt bestehend aus:
- Bestimmen in dem Fenster der Punkte des Bildes, deren Größe des Helligkeitsgradienten größer als ein Schwellwert ist, um die Innen- und Außenkonturen der Fassung in dem Fenster zu erhalten;
- Bestimmen der Form der Fassung in dem Fenster ausgehend von diesen Konturen;
- Bestimmen der Tangente an die Innenkontur der Fassung als Funktion dieses Fensters.

7. Verfahren nach Anspruch 6, dadurch gekennzeichnet, daß der Schwellwert derart gewählt wird, daß eine vorbestimmte Anzahl von Punkten des Bildes einen über dieser Schwelle liegenden Normwert des Gradienten darstellen.

8. Verfahren nach einem der Ansprüche 1 bis 7, dadurch gekennzeichnet, daß es einen Schritt des Bestimmens der Neigungswinkel umfaßt, umfassend die Schritte bestehend aus:
- Liefern von mindestens einer reellen Kontur der Fassung;
- Berechnen der Projektion dieser reellen Kontur auf das Bild für die verschiedenen, möglichen Werte der Neigungswinkel;
- Vergleichen dieser Projektion mit den dem Bild entnommenen Konturen, indem für diese Neigungswinkel die Korrelation zwischen dieser Projektion und den dem Bild entnommenen Konturen berechnet wird;
wobei die Neigungswinkel jene sind, für welche diese Korrelation maximal ist.

9. Verfahren nach einem der Ansprüche 1 bis 7, dadurch gekennzeichnet, daß es die Schritte umfaßt, bestehend aus:
- Liefern von mindestens einer reellen Kontur der Fassung und der Neigungswinkel der Fassung;
- Berechnen der Projektion der reellen Kontur auf das Bild als Funktion der Neigungswinkel;
- Vergleichen dieser Projektion mit den dem Bild entnommenen Werten, indem diese Projektion auf dem Bild abgetastet wird und indem in jeder Position dieser Projektion die Korrelation zwischen dieser Projektion und den dem Bild entnommenen Konturen berechnet wird;
- Bestimmen der Positionen der Tangenten an die Fassung bildenden, horizontalen und vertikalen Geraden ausgehend von der Position der Projektion, für welche die Korrelation maximal ist.

10. Verfahren nach einem der Ansprüche 1 bis 9, dadurch gekennzeichnet, daß der Schritt, welcher aus dem Berechnen ausgehend von diesen Positionen der Werte dieser metrologischen Parameter besteht, durch Vergleichen der relativen Positionen der Zentren der Pupillen und der Tangenten an die Innenkonturen der Fassung bildenden Geraden durchgeführt wird.
